# EUROPEAN PATENT SPECIFICATION

(11) **EP 4 401 808 B1**
(45) Date of publication and mention of the grant of the patent: **06.08.2025**
(21) Application number: 22785858.6
(22) Date of filing: 13.09.2022
(51) Int. Cl.: A61M 5/14, A61M 5/142, A61M 5/168

(54) **INFUSION PUMP AND NON-TRANSITORY MACHINE-READABLE STORAGE MEDIUM**
INFUSIONSPUMPE UND NICHT-ÜBERTRAGBARES MASCHINENLESBARES SPEICHERMEDIUM
POMPE D'INFUSION ET SUPPORT DE STOCKAGE NON TRANSITOIRE LISIBLE PAR UNE MACHINE

(30) Priority: 14.09.2021 US 202163244204 P
(43) Date of publication of application: 24.07.2024
(73) Proprietor: CareFusion 303, Inc., San Diego, CA 92130 (US)
(72) Inventor: WU, Richard S., San Diego, California 92130 (US)
(74) Representative: Zenz Patentanwälte Partnerschaft mbB
(86) International application number: PCT/US2022/043379
(87) International publication number: WO 2023/043744

(56) References cited:
- US-A1- 2005 145 010
- US-A1- 2018 099 089

## Description

### BACKGROUND

Downstream occlusion alarms frequently occur in a healthcare environment where infusion pumps are used. Moreover, over-infusion is a top complaint for infusion pump problems. US 2005/145010 Al discloses a system and method for determining when fluid is not flowing properly from a secondary infusion source during a secondary infusion. The system includes an upstream pressure sensor and a processor programmed to receive signals from the sensor and analyze the signals to determine if secondary fluid flow is proper. The processor samples the output signals from the upstream pressure sensor and analyzes the sampled signals to determine if a pressure rise in the infusion line has occurred when the secondary infusion is initiated. If a pressure rise, indicating that fluid from the secondary container has begun flowing into the infusion line, has not been detected, the processor is programmed to provide a signal indicating that attention should be given to the infusion set up.

### SUMMARY

The invention is defined in the independent claims. Preferable embodiments are further laid out in the dependent claims. According to various aspects of the subject technology, a method includes providing an infusion pump with one or more occluder valves comprising a lower occluder valve, the lower occluder valve configured to, when activated, cause a compression of a fluid tubing loaded into the infusion pump, the compression fluidically isolating a downstream portion of the fluid tubing from an upstream portion of the fluid tubing when the lower occluder valve is operating under a normal operating condition; placing the infusion pump in a default mode in which the lower occluder valve is activated; while in the default mode and the lower occluder valve is activated, with a lower pressure sensor, monitoring a pressure within the fluid tubing for a threshold deviation from an expected pressure; determining, before receiving an indication to start an infusion of a fluid through the fluid tubing, whether the threshold deviation was detected; configuring the infusion pump to start the infusion when the indication is received after the threshold deviation is detected; and providing a notification without starting the infusion when the indication is received and the threshold deviation is not detected. Other aspects include corresponding systems, apparatus, and computer program products for implementation of the corresponding method and its features.

According to various aspects of the subject technology, an infusion pump includes one or more occluder valves comprising a lower occluder valve, the lower occluder valve configured to, when activated, cause a compression of a fluid tubing loaded into the infusion pump, the compression fluidically isolating a downstream portion of the fluid tubing from an upstream portion of the fluid tubing when the lower occluder valve is operating under a normal operating condition; a lower pressure sensor downstream of the lower occluder valve; a processor configured to: place the infusion pump in a default mode in which the lower occluder valve is activated; while in the default mode and the lower occluder valve is activated, with the lower pressure sensor, monitor a pressure within the fluid tubing for a threshold deviation from an expected pressure; determine, before receiving an indication to start an infusion of a fluid through the fluid tubing, whether the threshold deviation was detected; configure the infusion pump to start the infusion when the indication is received after the threshold deviation is detected; and provide a notification without starting the infusion when the indication is received and the threshold deviation is not detected. Other aspects include corresponding systems, methods, and computer program products for implementation of the corresponding infusion pump.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description of Implementations below, in conjunction with the following drawings. Like reference numerals refer to corresponding parts throughout the figures and description.
FIG. 1 depicts an example infusion pump set-up, shown in use in its intended environment.
FIG. 2, an infusion pump is shown in perspective view with the front door open, showing the upstream fluid line and downstream fluid line in operative engagement with the pump.
FIG. 3 depicts an example configuration of a pump in a home position.
FIG. 4 depicts an example pressure signal response that deviates from an expected response.
FIG. 5 depicts an example configuration of a pump in a maintenance position.
FIG. 6 depicts an example maintenance workflow for diagnosing an infusion pump and/or its occluders.
FIGS. 7A and 7B depict an example clinician workflows for preventing hazardous operation of an infusion pump.
FIG. 8 depicts an example process for preventing hazardous operation of an infusion pump.
FIG. 9 is a conceptual diagram illustrating an example electronic system for preventing hazardous operation of an infusion pump.

### DETAILED DESCRIPTION

Reference will now be made to implementations, examples of which are illustrated in the accompanying drawings. In the following description, numerous specific details are set forth, in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

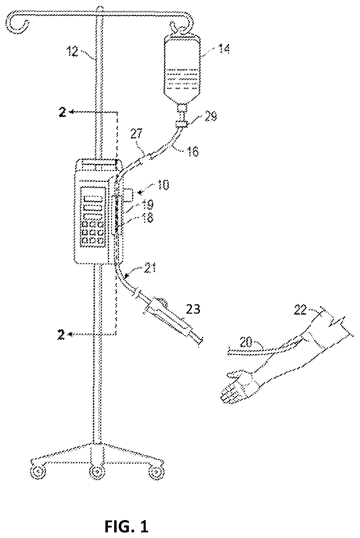

FIG. 1 depicts an example infusion pump set-up 10, shown in use in its intended environment, according to various aspects of the subject technology. In particular, the infusion pump set-up 10 is shown mounted to an intravenous (IV) pole 12 on which a fluid source 14 containing an IV fluid is held. The fluid source 14 is connected in fluid communication with an upstream fluid line 16. The fluid line 16 is a conventional IV infusion type tube typically used in a hospital or medical environment, and is made of any type of flexible tubing appropriate for use to infuse therapeutic fluids into a patient, such as polyvinylchloride (PVC). A flexible pumping fluid line 18 is mounted in operative engagement with a peristaltic pumping apparatus 19, for propelling fluid through a downstream fluid line 20, for example, to a patient's arm 22.

It will be understood by those skilled in the art that the upstream fluid line 16, the flexible line 18, and the downstream fluid line 20 may be portions of a continuous length of flexible tubing, with the portions defined by the location of the peristaltic pump 19. For convenience, the continuous length of flexible tubing is indicated by numeral 21. A roller clamp 23 (e.g., configured to provide for mechanical compression of the line to block the flow) may be positioned on the downstream fluid line 20 between the pump 10 and the patient's arm 22. In this context, the term "upstream" refers to that portion of the flexible tubing that extends between the fluid source and peristaltic pump, and the term "downstream" refers to that portion of the flexible tubing that extends from the peristaltic pump to the patient.

Also shown in FIG. 1 is a secondary administration setup generally indicated by numeral 24. The secondary administration setup 24 includes a secondary fluid container 25 that may be filled with a second therapeutic fluid for infusion into the patient 22. Fluid from the secondary fluid container 25 flows through a secondary fluid line 26 into the fluid line 16 through a connector 27. A manually operated valve 28 is located in the secondary line 26 to control the flow of fluid flowing out of the secondary container 25 into the upstream fluid line 16. The one-way check valve 29 is disposed in the upstream fluid line 16 between the primary fluid container 14 and the connector 27, the one-way check valve is configured so that when the elevation of the fluid in the secondary container 25 is greater than that of the primary container, the differential pressure within line 16 closes the check valve and prevents secondary fluid from flowing into the primary container 14, and also prevents fluid from flowing out of primary container 14. Thus, the check valve 29 generally prevents mixing of the primary and secondary infusion fluids.

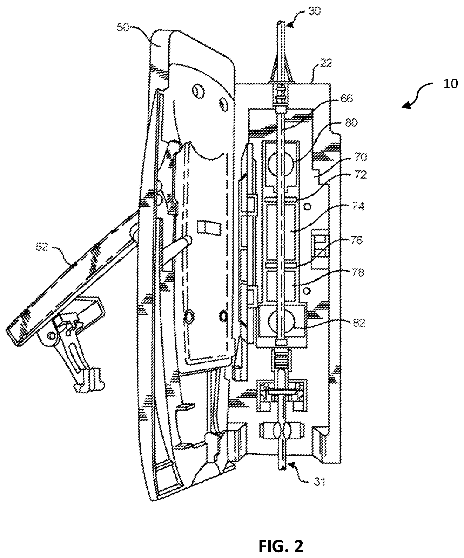

Turning now to FIG. 2, an infusion pump 10 is shown in perspective view with the front door 50 open, showing the upstream fluid line 30 and downstream fluid line 31 in operative engagement with the pump 10. The infusion pump 10 directly acts on a tube 66 that connects the upstream fluid line 30 to the downstream fluid line 31 to form a continuous fluid conduit, extending from the respective fluid supply 14 and/or 25 (FIG. 1) to the patient 22, 31, through which fluid is acted upon by the pump to move fluid downstream to the patient. Specifically, a pumping mechanism 70 acts as the flow control device of the pump to move fluid though the conduit. The upstream and downstream fluid lines and/or tube 66 may be coupled to a pump cassette or cartridge that is configured to be coupled to the pump 10, such EP 22 785 858.6 as the type described in co-pending U.S. patent application Ser. No. 13/827,775.

The type of pumping mechanism may vary and may be for example, a multiple finger pumping mechanism. For example, the pumping mechanism may be of the "four finger" type and includes an upstream occluding finger 72, a primary pumping finger 74, a downstream occluding finger 76, and a secondary pumping finger 78. The "four finger" pumping mechanism and mechanisms used in other linear peristaltic pumps operate by sequentially pressing on a segment of the fluid conduit by means of the cam-following pumping fingers and valve fingers 72, 74, 76, and 78. The pressure is applied in sequential locations of the conduit, beginning at the upstream end of the pumping mechanism and working toward the downstream end. At least one finger is always pressing hard enough to occlude the conduit. As a practical matter, one finger does not retract from occluding the tubing until the next one in sequence has already occluded the tubing; thus, at no time is there a direct fluid path from the fluid supply to the patient. The operation of peristaltic pumps including four finger pumps is well known to those skilled in the art and no further operational details are provided here. In this particular embodiment, FIG. 2 further shows a downstream pressure sensor 82 included in the pump 10 embodiment at a downstream location with respect to the pumping mechanism. The downstream pressure sensor 82 is mounted to the flow control device 70 and is located adjacent and downstream in relation to the flow control device. The downstream pressure sensor is located downstream from the flow control device, that is, at a location between the patient 22 (FIG. 1) and the flow control device, so that the connection of the correct fluid supply with the correct pump may be verified before any fluid is pumped to the patient.

With reference still to FIG. 2, an upstream pressure sensor 80 may also be included in the pump 10. The upstream pressure sensor is assigned to the flow control device or pumping mechanism 70 and, in this example, is further provided as an integral part of the pump 10. It is mounted to the flow control device 70 and is located adjacent and upstream in relation to the flow control device. The upstream pressure sensor is located upstream from the flow control device, that is, at a location between the fluid supply 14 and/or 25 (FIG. 1) and the flow control device, so that the connection of the correct fluid supply with the correct pump may be verified before any fluid is pumped to the patient.

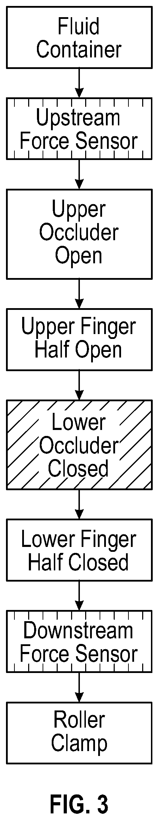

FIG. 3 depicts an example configuration of a pump 10 in a home position, according to various aspects of the subject technology. Before an infusion is started, when the pump is powered on, the occluders and the fingers of the pump are reset to default positions before the pump starts. In some implementations, these elements are reset every time the door is opened or closed, or when the pump is powered on or off, or when the pump is placed into a standby mode. When the elements are in their default positions the pump said to be in the "home" position. The home position is defined as the upper occluder being open, the upper finger being half open (e.g., between open and close), the lower occluder is closed (or shut), and the lower finger is half open.

Before an infusion is started, the clinician will typically connect (e.g., spike) the medication bag with a new administration set, at least partially fill a drip chamber, open the roller clamp, and prime the IV line. The clinician uses the roller clamp to control the priming rate, ensuring minimal spillage, and holds the distal end of the set with the other hand. Typically, the clinician closes the roller clamp once the administration set is fully primed. The clinician may then load the set into the pump and start programming the pump. During this step, the pump may be in the home position. The clinician may the open the roller clamp 23 and start the infusion. When the roller clamp is opened, fluid begins to move.

According to various implementations, a pump is programmed to detect upstream or downstream occlusions by way of its pressure sensors 80, 82, and to display and/or sound an alert when the occlusion is detected. In some instances, a downstream occlusion alarm may occur if the clinician forgets to open the roller clamp before starting the infusion.

The subject technology provides a hardware-software mechanism that prevents unnecessary alarms by guiding the clinician through a workflow when starting an infusion. In this regard, the subject technology prevents unnecessary downstream occlusion alarms due to, for example, the failure to open a roller clamp.

Before the infusion is started, when a clinician opens the roller clamp, there will be an occlusion in the line because the lower occluder is closed. A backpressure may pump fluid upwards against the closed lower occluder. This backpressure will cause a deviation in a signal seen by the downstream pressure sensor that is greater than a slope of an expected signal (which may be negligible).

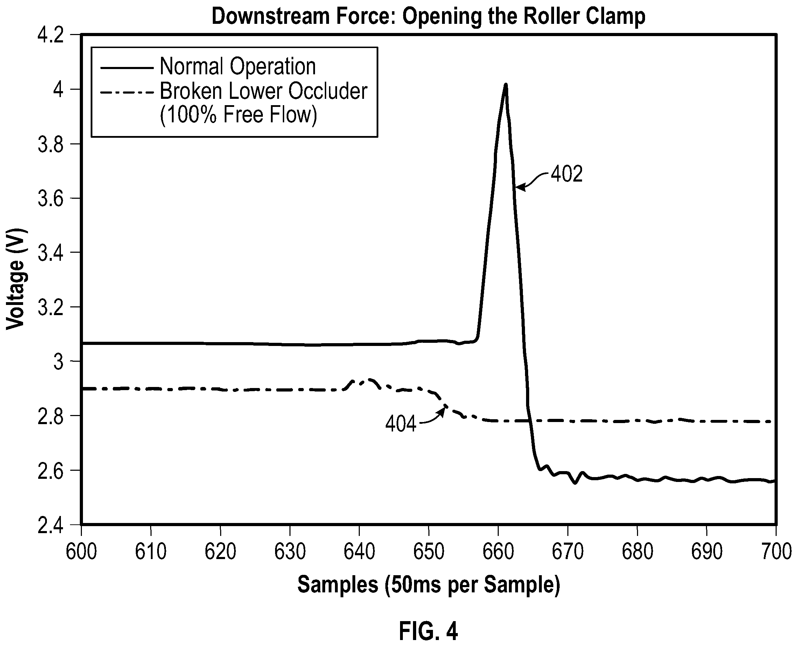

FIG. 4 depicts an example pressure signal response that deviates from an expected response, according to aspects of the subject technology. **In** various implementations, the downstream pressure sensor 82 of the pump provides a signal representative of a pressure within the tubing. When there is an occlusion in the line, the signal may exhibit a force spike 402 because the fluid has nowhere to go and so the tubing here expands, and a pressure is created in the tube. The pressure sensor 82 will see a voltage spike 402, as depicted in FIG. 3. The spike is indicative of everything is working correctly. If the spike is not detected, then the pump may provide an alarm or prevent further operations until the roller clamp 23 is opened. Accordingly, the pump 10 may utilize the roller clamp pressure profile (which originates from the roller clamp outside the pump) to detect whether it is ok to start an infusion.

The downstream (and upstream) pressure sensor may (e.g., when the infusion pump is turned on and/or activated), constantly monitor pressure within the infusion line 20 and translate the measured pressure reading to a voltage. This voltage is then provided (e.g., by analog-digital converters within the pump) to a processor. The processor may then determine whether the force spike occurred based on whether the voltage value readings received satisfy a threshold. **In** some implementations, the threshold is a (positive or negative) threshold voltage which must be exceeded. **In** some implementations, the processor monitors readings for a moving window of time. Satisfaction of the threshold may include, for example, an average or mean of the values measured during the window of time satisfying (e.g., meeting and/or exceeding) a threshold value, or a minimum or a maximum value within that window satisfying the threshold value.

In some implementations, the force deviating from an expected result may include detecting a (e.g. large) positive or negative slope in the measured values that is greater than an expected slope. The processor may continuously determine a slope of the voltage readings (e.g., within the window), and the threshold is a threshold slope that, when met and/or exceeded, triggers detection of the force spike. In some implementations, determination of the slope involves a 2-point slope calculation. The slope may be negative or positive.

In various implementations of the subject technology, if the pump doesn't see this force spike before an infusion happens, then the pump may determine that the clinician forgot to open the roller clamp. In other words, the pump monitors the pressure sensor for the signal, expecting to see the voltage spike, and if it does not detect the spike before the clinician when the infusion is started then the pump provides an alarm and/or displays a warning message.

The clinician loads the set into the pump and closes the door. Once the door closes, the detection algorithm activates and begins to constantly monitor for the force spike. A potential occlusion hazard arises when the clinician does not open the roller clamp, yet starts the pump. In this regard, the active compression by the roller clamp on the downstream fluid line 20 may cause an occlusion, thereby preventing the fluid within the tube to flow and further causing the infusion pump to trigger an alarm and/or terminate the infusion.

The downstream pressure sensor 82, used to determine whether the roller clamp was properly opened, may also be used to determine whether the lower occluder is properly functioning. A leaking occluder valve may lead to an over infusion of the medication. There is a need to verify the proper operation of LVP Infusion pumps with regards to leakage of the occluder valves in pumps. Accordingly, the hardware-software mechanism of the subject technology also identifies whether the lower occluder valve is leaking.

When the infusion device is in the home position, the lower occluder is supposed to be closed which creates the previously described occlusion. However, if there is damage on this lower occluder then the force spike may not be detected. The springs which force the lower occluder downward against the tubing may wear out to the extent that the lower occluder may not close all the way. In an extreme case, if the lower occluder you know doesn't close at all, for example because it is completely broken and doesn't close at all, then no force spike will be detected. Instead, the pressure sensor will measure a (relatively) flat line 404, such as that depicted in FIG. 4.

If the lower occluder is broken, then some of the pressure force may trigger an upper force spike at the upstream pressure sensor 80. In this regard, in some implementations, the processor may (e.g., continuously) measure both the upstream pressure sensor 80 and the downstream pressure sensor 82. The measurements may be compared, for example, by subtracting one reading from the other to see if the foregoing threshold has been reached. In some implementations, an upper force spike detected by the upper pressure sensor 80 may be indicative of a problem with the lower (closed) occluder, regardless of whether a spike is seen at the lower pressure sensor 82. In one example, if an upstream spike is detected but not a downstream spike, the processor may provide an alert for display on the infusion device to check the downstream occluder.

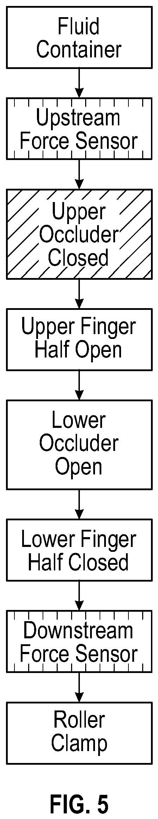

FIG. 5 depicts an example configuration of a pump 10 in a maintenance position, according to various aspects of the subject technology. In some implementations, the pump 10 may include a control to switch the pump into a maintenance mode. This control may be made available to authorized individuals for maintenance of the pump 10. In this mode, a further control may allow a technician to switch the occluder positions. For example, the upper occluder may close and the lower occluder may open, as depicted in FIG. 5.

During a maintenance check, the fluid tubing 20 may be primed with fluid, the roller clamp closed, and the pump switched into maintenance mode. Then the processor of the pump may begin monitoring for a lower force spike, as previously described above. Additionally, or in the alternative, the occluder positions may be switched, and the roller clamp may be opened to determine whether a force spike is detected by the upper pressure sensor 80. If the upper force spike is detected, then an alert may be provided indicating to check the upper occluder and/or that the upper occluder may be damaged or defective.

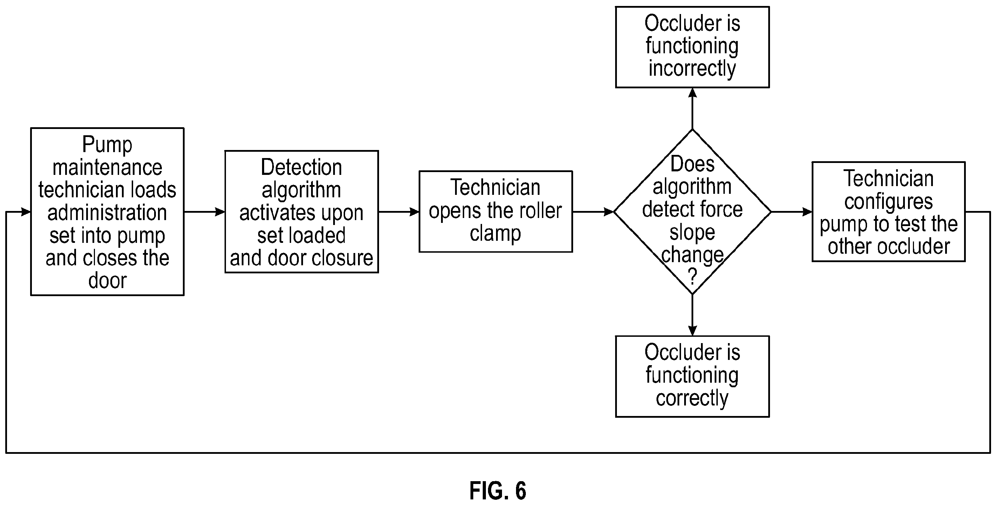

FIG. 6 depicts an example maintenance workflow for diagnosing an infusion pump 10 and/or its occluders, according to various aspects of the subject technology. A pump maintenance technician loads an administration set into the pump 10 and closes the door 50. The pump is then set to the maintenance mode, as described with regard to FIG. 5. The detection algorithm activates upon the set being loaded and the door closure. The technician then proceeds to open the roller clamp 23. If the processor (and algorithm) detects the force spike then the occluder may be presumed to be functioning correctly, and a notification may be displayed to indicate normal operation. If the processor does not detect the force spike, then an alert may be displayed that the occluder is faulty. The technician may then proceed to configure the pump, as described with regard to FIG. 5, to test the other occluder.

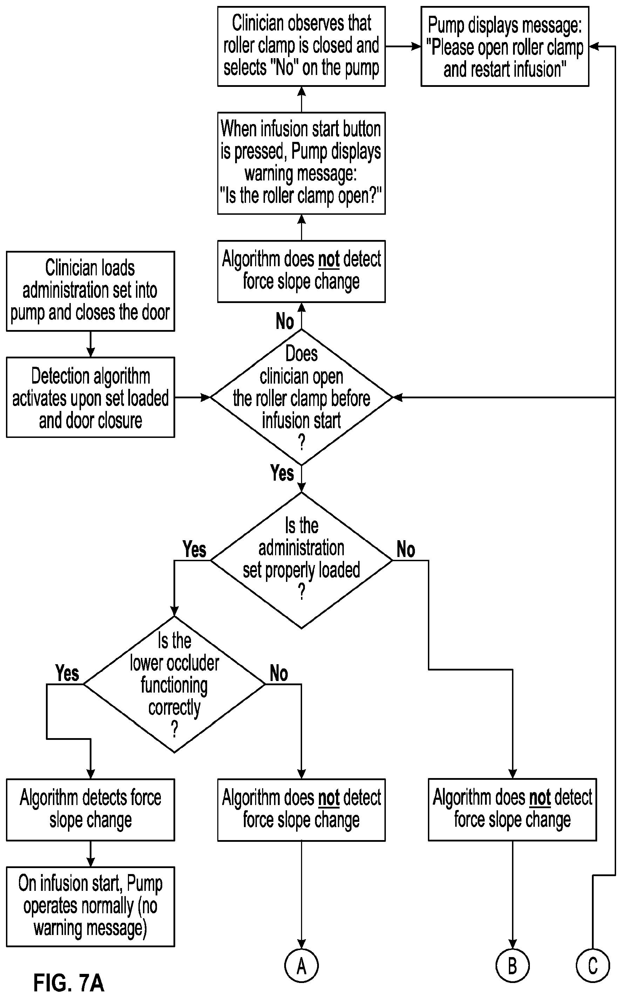

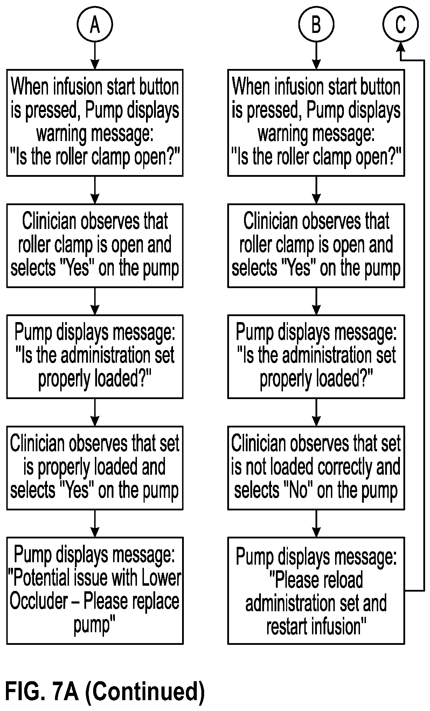

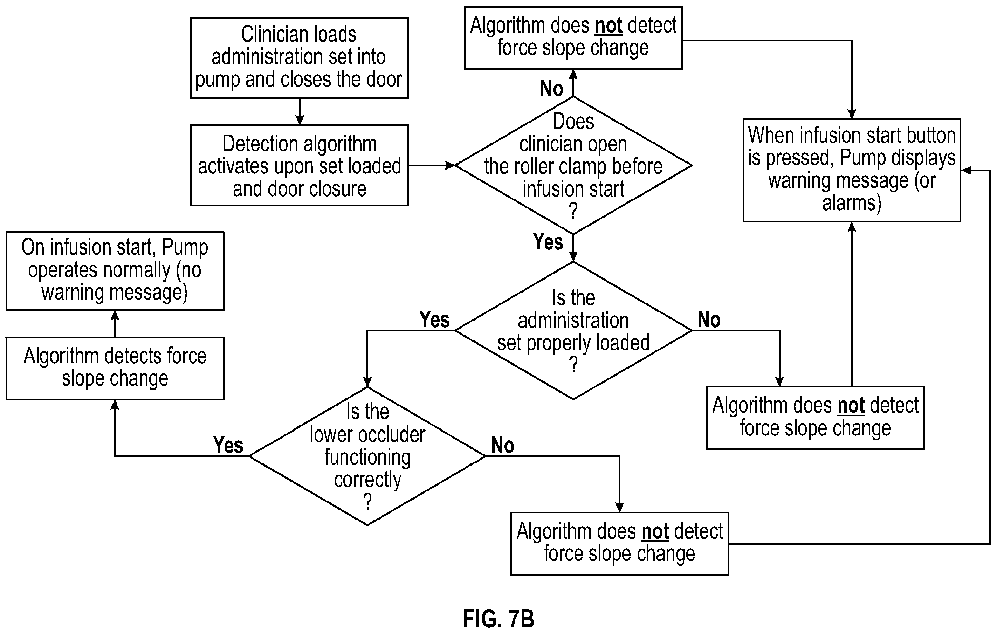

FIGS. 7A and 7B depict a example clinician workflows for preventing hazardous operation of an infusion pump 10, according to various aspects of the subject technology. In the depicted example, the clinician loads a fluid administration set into the pump 10 and closes the door 50. The processor activates the detection algorithm upon the set being loaded and/or the door being closed. If the clinician does not open the roller clamp 23 before the infusion is started then, as described previously, a force spike will not be detected. In some implementations, a warning message will be displayed on the clinician starting the infusion pump 10 (e.g., when the clinician presses the start button).

The warning message may include asking whether the roller clamp 23 was opened. If the clinician confirms that the roller clamp 23 was not opened (remains closed) then the pump 10 may instruct the clinician to open the roller clamp and restart the infusion. If the clinician confirms that the roller clamp was indeed opened, and a force spike has not been detected then the pump 10 may display a message asking whether the administration set was loaded properly. If the clinician answers that the administration set was not loaded properly then the pump 10 may request reloading of the set and the process may restart. Additionally, or in the alternative, the pump 10 may recycle the prompts as depicted in FIG. 7A.

If the clinician confirms proper loading of the set and a force spike is still not detected, then the pump 10 may determine that the lower occluder valve is not functioning correctly. A message may be displayed by the pump 10 that there is a potential issue with the lower occluder and instruct the clinician to replace the pump or flag it for maintenance.

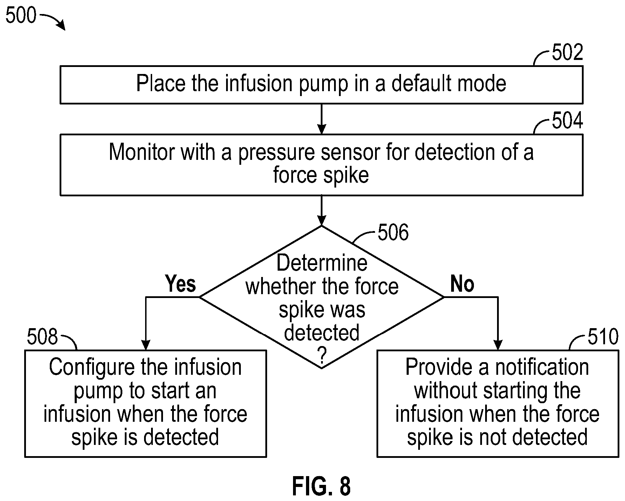

FIG. 8 depicts an example process 500 for preventing hazardous operation of an infusion pump, according to aspects of the subject technology. For explanatory purposes, the various blocks of example process 500 are described herein with reference to FIGS. 1-7, and the components and/or processes described herein. The one or more of the blocks of process 500 may be implemented, for example, by one or more computing devices including, for example, pump 10. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 500 are described as occurring in serial, or linearly. However, multiple blocks of example process 500 may occur in parallel. In addition, the blocks of example process 500 need not be performed in the order shown and/or one or more of the blocks of example process 500 need not be performed.

An infusion pump is provided with one or more occluder valves comprising a lower occluder valve. The lower occluder valve is configured to, when activated, cause a compression of a fluid tubing loaded into the infusion pump. When the pump element is operating under a normal operating condition, the compression fluidically isolates a downstream portion of the fluid tubing from an upstream portion of the fluid tubing.

In the depicted example, the infusion pump is placed in a default mode in which the lower occluder valve is activated (502). While in the default mode and the lower occluder valve is activated, with a lower pressure sensor 82, the processor monitors a pressure within the fluid tubing for a threshold deviation from an expected pressure (804). As described previously, the processor may be monitoring to detect a force spike in the signal provided from the lower pressure sensor 82 (a "lower force spike").

With brief reference to FIG. 2, the lower pressure sensor 82 is downstream of the lower occluder valve. As described previously, the force spike may be caused by opening a roller clamp downstream of the infusion pump to open a fluid path between the infusion pump and a patient to which the fluid tubing is fluidically connected for infusion of a fluid by the infusion pump. In this regard, the opening of the roller clamp may occur after the fluid tubing has been primed with the fluid prior to prior to the infusion pump being placed in the default mode.

The processor determines, before receiving an indication to start an infusion of a fluid through the fluid tubing, whether the lower force spike was detected (506). This determination may be made on activation of an infusion start control or, in some implementations, the start control may be provided upon the fore spike being detected. According to various implementations, the lower force spike may include a voltage spike in a plurality of voltage readings over a period of time. For example, the upper and lower pressure sensors 80, 82 may continuously monitor pressure and the voltage readings may be values measured within a moving window of time.

The processor then configures the infusion pump to start the infusion when the lower force spike is detected (508), and provides a notification without starting the infusion when the lower force spike is not detected (510). Accordingly, hazardous operation of an infusion pump is prevented.

In some implementations, a slope of the voltage readings is determined (e.g., in the moving window) and if the slope satisfies a threshold slope then a force spike is determined to occur. In some implementations, a force spike is determined to occur when at least one reading of the plurality of the voltage readings satisfies a threshold voltage.

In some implementations, the processor receives the indication to start the infusion of the fluid through the fluid tubing and configures the infusion pump to start the infusion based on receiving the indication. In some implementations, configuring the infusion pump to start the infusion may include, for example, presenting, for display on a display screen associated with the infusion pump, a graphical start control that, when activated, causes the infusion to start, wherein the graphical start control is not displayed until the lower force spike is detected. For example, the pump 10 may hid the option to start until the pump detects the spike. At that point a soft key may be presented on the pump for initiating the infusion programmed into the pump.

In some implementations, the notification instructs a user to check a roller clamp coupled to the fluid tubing, for example, to see whether the roller clamp is the cause of an occlusion in the tubing. The processor may then receive an indication (e.g., from a user via the pump's input) that the roller clamp is open. In some instances, particularly when a force spike has not been detected, the confirmation may provide an indication that the roller clamp is not the issue. The pump may then request that the user check placement of the fluid tubing in the infusion pump. Upon receiving confirmation of the placement of the fluid tubing (e.g., that it is correctly loaded in the pump), the processor may provide an alert regarding the lower occluder valve. For example, the pump may indicate that the lower occluder valve may be faulty, and the pump should be serviced. In some implementations, the pump is locked and prevented from being serviced until it is authorized to return to service by a maintenance technician.

In some implementations, while in the default mode, the processor (e.g., executing the algorithm) may also monitor an upper pressure sensor 80 of the infusion device for an upper force spike. In this regard, the upper pressure sensor 80 may be positioned upstream of an upper occluder valve. The processor determines, based on receiving the indication to start the infusion, whether the upper force spike was detected. If the upper force spike is detected, then an indication may be provided that the lower occluder valve is not functioning. This is because, in the default mode, the lower occluder valve is supposed to be closed while the upper occluder valve is expected to be open. If the lower occluder valve is faulty and cannot fully compress the tubing, then the fluid may backflow to and past the upper occluder valve where it may be detected by the upper pressure sensor 80.

As described previously, the infusion pump 10 may include a maintenance mode. Upon receiving an indication to enter a maintenance mode, the lower occluder valve may be deactivated while an upper occluder of the one or more occluder valves is activated. Under normal circumstances, the upper occluder being activated causes a compression of the fluid tubing at the upper occluder. The process 500 continues by the processor receiving an indication that the fluid tubing is primed with the fluid. This may be by way of a clinician confirming the priming via an input on the pump. Additionally, or in the alternative, the pump 10 may include a control for initiating the priming which, when activated, performs the priming and includes the indication. In the same manner, the pump 10 may receive an indication that a roller clamp downstream of the pump is opened. After the roller clamp is opened, the pump 10 may determine whether an upper force spike is detected by the upper pressure sensor 80, upstream of the upper occluder, and provide a notification regarding failure of the upstream occluder valve when the upper force spike is detected.

Many of the above-described example process 500, and related features and applications, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

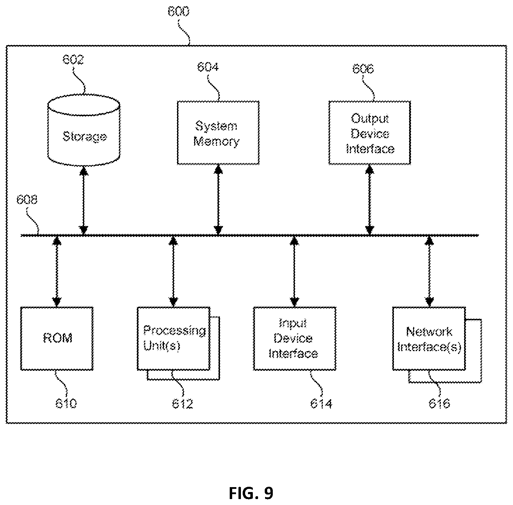

FIG. 9 is a conceptual diagram illustrating an example electronic system 600 for preventing hazardous operation of an infusion pump, according to aspects of the subject technology. Electronic system 600 may be a computing device for execution of software associated with one or more portions or steps of method 500, or components and methods provided by FIGS. 1-8, including but not limited to computing hardware within patient care device 12, or syringe pump 200, and/or any computing devices or associated terminals disclosed herein. In this regard, electronic system 600 may include the infusion pump 10, a computing device within or connected to the infusion pump 10.

Electronic system 600 may include various types of computer readable media and interfaces for various other types of computer readable media. In the depicted example, electronic system 600 includes a bus 608, processing unit(s) 612, a system memory 604, a read-only memory (ROM) 610, a permanent storage device 602, an input device interface 614, an output device interface 606, and one or more network interfaces 616. In some implementations, electronic system 600 may include or be integrated with other computing devices or circuitry for operation of the various components and methods previously described.

Bus 608 collectively represents all system, peripheral, and chipset buses that communicatively connect the numerous internal devices of electronic system 600. For instance, bus 408 communicatively connects processing unit(s) 612 with ROM 610, system memory 604, and permanent storage device 602.

From these various memory units, processing unit(s) 612 retrieves instructions to execute and data to process, in order to execute the processes of the subject disclosure. The processing unit(s) can be a single processor or a multi-core processor in different implementations.

ROM 610 stores static data and instructions that are needed by processing unit(s) 612 and other modules of the electronic system. Permanent storage device 602, on the other hand, is a read-and-write memory device. This device is a non-volatile memory unit that stores instructions and data even when electronic system 600 is off. Some implementations of the subject disclosure use a mass-storage device (such as a magnetic or optical disk and its corresponding disk drive) as permanent storage device 602.

Other implementations use a removable storage device (such as a floppy disk, flash drive, and its corresponding disk drive) as permanent storage device 602. Like permanent storage device 602, system memory 604 is a read-and-write memory device. However, unlike storage device 602, system memory 604 is a volatile read-and-write memory, such as random access memory. System memory 604 stores some of the instructions and data that the processor needs at runtime. In some implementations, the processes of the subject disclosure are stored in system memory 604, permanent storage device 602, and/or ROM 610. From these various memory units, processing unit(s) 612 retrieves instructions to execute and data to process, in order to execute the processes of some implementations.

Bus 608 also connects to input and output device interfaces 614 and 606. Input device interface 614 enables the user to communicate information and select commands to the electronic system. Input devices used with input device interface 614 include, e.g., alphanumeric keyboards and pointing devices (also called "cursor control devices"). Output device interfaces 606 enables, e.g., the display of images generated by the electronic system 600. Output devices used with output device interface 606 include, e.g., printers and display devices, such as cathode ray tubes (CRT) or liquid crystal displays (LCD). Some implementations include devices such as a touchscreen that functions as both input and output devices.

Also, as shown in FIG. 6, bus 608 also couples electronic system 600 to a network (not shown) through network interfaces 616. Network interfaces 616 may include, e.g., a wireless access point (e.g., Bluetooth or WiFi) or radio circuitry for connecting to a wireless access point. Network interfaces 616 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 600 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software, depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

### Further Consideration:

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa.

Headings and subheadings, if any, are used for convenience only.

The predicate words "configured to", "operable to", and "programmed to" do not imply any particular tangible or intangible modification of a subject, but, rather, are intended to be used interchangeably. For example, a processor configured to monitor and control an operation or a component, may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An implementation may provide one or more examples. A phrase such as an "implementation" may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine-readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In some implementations, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.
and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. A non-transitory machine-readable storage medium storing instructions thereon that, when executed by one or more processing units, perform a process, comprising:
operating an infusion pump (10) with one or more occluder valves comprising a lower occluder valve, the lower occluder valve configured to, when activated, cause a compression of a fluid tubing (20) loaded into the infusion pump (10), the compression fluidically isolating a downstream portion of the fluid tubing from an upstream portion of the fluid tubing when the lower occluder valve is operating under a normal operating condition;
placing the infusion pump (10) in a default mode in which the lower occluder valve is activated;
while in the default mode and the lower occluder valve is activated, with a lower pressure sensor (82), monitoring a pressure within the fluid tubing for a threshold deviation from an expected pressure;
determining, before receiving an indication to start an infusion of a fluid through the fluid tubing (20), whether the threshold deviation was detected;
configuring the infusion pump (10) to start the infusion when the indication is received after the threshold deviation is detected; and
providing a notification without starting the infusion when the indication is received and the threshold deviation is not detected.

2. The non-transitory machine-readable storage medium of Claim 1, the process further comprising:
receiving the indication to start the infusion of the fluid through the fluid tubing; and
configuring the infusion pump to start the infusion based on receiving the indication.

3. The non-transitory machine-readable storage medium of Claim 1 or Claim 2, wherein configuring the infusion pump to start the infusion comprises:
presenting, for display on a display screen associated with the infusion pump, a graphical start control that, when activated, causes the infusion to start, wherein the graphical start control is not displayed until the threshold deviation is detected.

4. The non-transitory machine-readable storage medium of any one of Claims 1 through 3, wherein the threshold deviation comprises a voltage spike in a plurality of voltage readings over a period of time.

5. The non-transitory machine-readable storage medium of any one of Claims 1 through 4, wherein monitoring the pressure for the threshold deviation comprises:
determining a slope of the voltage readings; and
determining that the slope satisfies a threshold slope.

6. The non-transitory machine-readable storage medium of any one of Claims 1 through 4, wherein monitoring the pressure for the threshold deviation comprises:
determining at least one reading of the plurality of the voltage readings satisfies a threshold voltage.

7. The non-transitory machine-readable storage medium of any one of Claims 1 through 6, the process further comprising:
while in default mode, monitoring an upper pressure sensor of the infusion pump for an upper pressure deviation in an upstream portion of the fluid tubing, the upper pressure sensor being upstream of an upper occluder valve of the one or more occluder valves; and
determining, based on receiving the indication to start the infusion, whether the upper pressure deviation was detected;
providing an indication that the lower occluder valve is not functioning when the upper pressure deviation is detected after receiving the indication to start the infusion.

8. The non-transitory machine-readable storage medium of any one of Claims 1 through 7, wherein the notification indicates an occlusion in the fluid tubing.

9. The non-transitory machine-readable storage medium of any one of Claims 1 through 8, wherein the notification instructs a user to check a roller clamp coupled to the fluid tubing.

10. The non-transitory machine-readable storage medium of Claim 9, the process further comprising:
receiving an indication that the roller clamp is open;
providing a request to check placement of the fluid tubing in the infusion pump;
receiving confirmation of the placement of the fluid tubing; and
providing, based on receiving the confirmation, an alert regarding the lower occluder valve.

11. The non-transitory machine-readable storage medium of any one of Claims 1 through 8, the process further comprising:
receiving an indication to enter a maintenance mode in which the lower occluder valve is deactivated and an upper occluder valve of the one or more occluder valves is activated, the upper occluder valve being activated comprises causing a compression of the fluid tubing at the upper occluder valve;
receiving an indication that the fluid tubing is primed with the fluid;
receiving an indication that a roller clamp downstream of the infusion pump is opened;
determining, after the roller clamp is opened, whether an upper pressure deviation is detected by an upper pressure sensor, upstream of the upper occluder valve; and
providing a notification regarding failure of the upper occluder valve when the upper pressure deviation is detected.

12. The non-transitory machine-readable storage medium of any one of Claims 1 through 8, wherein the threshold deviation is caused by opening a roller clamp downstream of the infusion pump to open a fluid path between the infusion pump and a patient to which the fluid tubing is fluidically connected for infusion of a fluid by the infusion pump, the opening of the roller clamp occurring after the fluid tubing has been primed with the fluid prior to prior to the infusion pump being placed in the default mode.

13. An infusion pump (10), comprising:
one or more occluder valves comprising a lower occluder valve, the lower occluder valve configured to, when activated, cause a compression of a fluid tubing (20) loaded into the infusion pump (10), the compression fluidically isolating a downstream portion of the fluid tubing from an upstream portion of the fluid tubing when the lower occluder valve is operating under a normal operating condition;
a lower pressure sensor (82) downstream of the lower occluder valve;
a processor configured to:
place the infusion pump (10) in a default mode in which the lower occluder valve is activated;
while in the default mode and the lower occluder valve is activated, with the lower pressure sensor, monitor a pressure within the fluid tubing for a threshold deviation from an expected pressure;
determine, before receiving an indication to start an infusion of a fluid through the fluid tubing, whether the threshold deviation was detected;
configure the infusion pump to start the infusion when the indication is received after the threshold deviation is detected; and
provide a notification without starting the infusion when the indication is received and the threshold deviation is not detected.

14. The infusion pump of Claim 13, wherein the processor is further configured to:
receive the indication to start the infusion of the fluid through the fluid tubing; and
configure the infusion pump to start the infusion based on receiving the indication.

15. The infusion pump of Claim 13 or Claim 14, wherein configuring the infusion pump to start the infusion comprises:
presenting, for display on a display screen associated with the infusion pump, a graphical start control that, when activated, causes the infusion to start, wherein the graphical start control is not displayed until the threshold deviation is detected.

16. The infusion pump of any one of Claims 13 through 15, wherein the threshold deviation comprises a voltage spike in a plurality of voltage readings over a period of time.

17. The infusion pump of any one of Claims 13 through 15, wherein monitoring the pressure for the threshold deviation comprises:
determining a slope of the voltage readings; and
determining that the slope satisfies a threshold slope.

18. The infusion pump of any one of Claims 13 through 17, wherein the processor is further configured to:
while in default mode, monitoring an upper pressure sensor of the infusion pump for an upper pressure deviation in an upstream portion of the fluid tubing, the upper pressure sensor being upstream of an upper occluder valve of the one or more occluder valves; and
determining, based on receiving the indication to start the infusion, whether the upper pressure deviation was detected;
providing an indication that the lower occluder valve is not functioning when the upper pressure deviation is detected after receiving the indication to start the infusion.

19. The infusion pump of Claim 18, wherein the notification instructs a user to check a roller clamp coupled to the fluid tubing, and wherein the processor is further configured to:
receiving an indication that the roller clamp is open;
providing a request to check placement of the fluid tubing in the infusion pump;
receiving confirmation of the placement of the fluid tubing; and
providing, based on receiving the confirmation, an alert regarding the lower occluder valve.

20. The infusion pump of Claim 18, wherein the processor is further configured to:
receiving an indication to enter a maintenance mode in which the lower occluder valve is deactivated and an upper occluder of the one or more occluder valves is activated, the upper occluder being activated comprises causing a compression of the fluid tubing at the upper occluder;
receiving an indication that the fluid tubing is primed with the fluid;
receiving an indication that a roller clamp downstream of the infusion pump is opened;
determining, after the roller clamp is opened, whether an upper pressure deviation is detected by an upper pressure sensor, upstream of the upper occluder; and
providing a notification regarding failure of the upper occluder valve when the upper pressure deviation is detected.

## Patentansprüche

1. Ein nicht flüchtiges, maschinenlesbares Speichermedium, das Anweisungen speichert, die, wenn sie von einer oder mehreren Verarbeitungseinheiten ausgeführt werden, ein Verfahren ausführen, umfassend:
Betreiben einer Infusionspumpe (10) mit einem oder mehreren Okklusionsventilen, die ein unteres Okklusionsventil umfassen, wobei das untere Okklusionsventil so konfiguriert ist, dass es bei Aktivierung eine Kompression eines in die Infusionspumpe (10) eingebrachten Fluidschlauchs (20) bewirkt, wobei die Kompression einen stromabwärts gelegenen Abschnitt des Fluidschlauchs von einem stromaufwärts gelegenen Abschnitt des Fluidschlauchs fluidisch isoliert, wenn das untere Okklusionsventil unter einer normalen Betriebsbedingung arbeitet;
Anordnen der Infusionspumpe (10) in einem Standardmodus, in dem das untere Okklusionsventil aktiviert ist;
während sich die Infusionspumpe (10) im Standardmodus befindet und das untere Okklusionsventil aktiviert ist, Überwachen eines Drucks innerhalb des Fluidschlauchs mit einem unteren Drucksensor (82) auf eine Schwellenwertabweichung von einem erwarteten Druck;
Bestimmen, bevor ein Signal zum Starten einer Infusion eines Fluids durch den Fluidschlauch (20) empfangen wird, ob die Schwellenwertabweichung erfasst wurde;
Konfigurieren der Infusionspumpe (10) zum Starten der Infusion, wenn das Signal empfangen wird, nachdem die Schwellenwertabweichung erfasst wurde; und
Bereitstellen einer Benachrichtigung, ohne die Infusion zu starten, wenn das Signal empfangen wird und die Schwellenwertabweichung nicht erfasst wird.

2. Ein nicht flüchtiges maschinenlesbares Speichermedium nach Anspruch 1, wobei das Verfahren ferner umfasst:
Empfangen des Signals zum Starten der Infusion des Fluids durch den Fluidschlauch; und
Konfigurieren der Infusionspumpe zum Starten der Infusion auf der Grundlage des Empfangens des Signals.

3. Ein nicht flüchtiges maschinenlesbares Speichermedium nach Anspruch 1 oder Anspruch 2, wobei das Konfigurieren der Infusionspumpe zum Starten der Infusion umfasst:
Anzeigen einer grafischen Startsteuerung auf einem mit der Infusionspumpe verbundenen Bildschirm, die bei Aktivierung die Infusion startet, wobei die grafische Startsteuerung erst angezeigt wird, wenn die Schwellenwertabweichung erkannt wird.

4. Ein nicht flüchtiges maschinenlesbares Speichermedium nach einem der Ansprüche 1 bis 3, wobei die Schwellenwertabweichung einen Spannungsspitzenwert in einer Vielzahl von Spannungsmesswerten über einen Zeitraum umfasst.

5. Ein nicht flüchtiges, maschinenlesbares Speichermedium nach einem der Ansprüche 1 bis 4, wobei das Überwachen des Drucks auf die Schwellenwertabweichung umfasst:
Bestimmen einer Steigung der Spannungsmesswerte; und
Bestimmen, dass die Steigung einen Schwellenwert für die Steigung erfüllt.

6. Ein nicht flüchtiges maschinenlesbares Speichermedium nach einem der Ansprüche 1 bis 4, wobei das Überwachen des Drucks auf die Schwellenwertabweichung umfasst:
Bestimmen, dass mindestens ein Messwert der mehreren Spannungsmesswerte eine Schwellenspannung erfüllt.

7. Ein nicht flüchtiges maschinenlesbares Speichermedium nach einem der Ansprüche 1 bis 6, wobei das Verfahren ferner umfasst:
während sich das System im Fehlermodus befindet, Überwachen eines oberen Drucksensors der Infusionspumpe auf eine obere Druckabweichung in einem stromaufwärts gelegenen Abschnitt des Fluidschlauchs, wobei der obere Drucksensor stromaufwärts von einem oberen Okklusionsventil des einen oder der mehreren Okklusionsventile angeordnet ist; und
Bestimmen auf der Grundlage des Empfangens des Signals zum Starten der Infusion, ob die obere Druckabweichung erfasst wurde;
Bereitstellen eines Signals, dass das untere Okklusionsventil nicht funktioniert, wenn die obere Druckabweichung nach Empfang des Signals zum Starten der Infusion erfasst wird.

8. Ein nicht flüchtiges maschinenlesbares Speichermedium nach einem der Ansprüche 1 bis 7, wobei die Benachrichtigung eine Okklusion im Fluidschlauch anzeigt.

9. Ein nicht flüchtiges, maschinenlesbares Speichermedium nach einem der Ansprüche 1 bis 8, wobei die Benachrichtigung einen Benutzer anweist, eine mit dem Fluidschlauch verbundene Rollenklemme zu überprüfen.

10. Ein nicht flüchtiges maschinenlesbares Speichermedium nach Anspruch 9, wobei das Verfahren ferner umfasst:
Empfangen eines Signals, dass die Rollenklemme offen ist;
Bereitstellen einer Aufforderung, die Platzierung des Fluidschlauchs in der Infusionspumpe zu überprüfen;
Empfangen einer Bestätigung der Platzierung des Fluidschlauchs; und
Bereitstellen einer Warnung bezüglich des unteren Okklusionsventils auf der Grundlage des Empfangens der Bestätigung.

11. Ein nicht flüchtiges, maschinenlesbares Speichermedium nach einem der Ansprüche 1 bis 8, wobei das Verfahren ferner umfasst:
Empfangen eines Signals zum Aufrufen eines Wartungsmodus, in dem das untere Okklusionsventil deaktiviert und ein oberes Okklusionsventil des einen oder der mehreren Okklusionsventile aktiviert wird, wobei das Aktivieren des oberen Okklusionsventils ein Zusammendrücken des Fluidschlauchs am oberen Okklusionsventil umfasst;
Empfangen eines Signals, dass der Fluidschlauch mit dem Fluid gefüllt ist;
Empfangen eines Signals, dass eine Rollenklemme stromabwärts der Infusionspumpe geöffnet ist;
Bestimmen, nachdem die Rollenklemme geöffnet ist, ob eine obere Druckabweichung durch einen oberen Drucksensor stromaufwärts des oberen Okklusionsventils erfasst wird; und
Bereitstellen einer Benachrichtigung bezüglich eines Ausfalls des oberen Okklusionsventils, wenn die obere Druckabweichung erfasst wird.

12. Ein nicht flüchtiges, maschinenlesbares Speichermedium nach einem der Ansprüche 1 bis 8, wobei die Schwellenwertabweichung dadurch verursacht wird, dass eine Rollenklemme stromabwärts der Infusionspumpe geöffnet wird, um einen Fluidweg zwischen der Infusionspumpe und einem Patienten zu öffnen, mit dem der Fluidschlauch stömungstechnisch verbunden ist, um ein Fluid durch die Infusionspumpe zu infundieren, wobei das Öffnen der Rollenklemme erfolgt, nachdem der Fluidschlauch mit dem Fluid gefüllt wurde, bevor die Infusionspumpe in den Standardmodus versetzt wurde.

13. Eine Infusionspumpe (10), umfassend:
ein oder mehrere Okklusionsventile, die ein unteres Okklusionsventil umfassen, wobei das untere Okklusionsventil so konfiguriert ist, dass es bei Aktivierung eine Kompression eines in die Infusionspumpe (10) eingebrachten Fluidschlauchs (20) bewirkt, wobei die Kompression einen stromabwärts gelegenen Abschnitt des Fluidschlauchs von einem stromaufwärts gelegenen Abschnitt des Fluidschlauchs fluidisch isoliert, wenn das untere Okklusionsventil unter einer normalen Betriebsbedingung arbeitet;
einen Unterdrucksensor (82) stromabwärts des unteren Okklusionsventils;
einen Prozessor, der so konfiguriert ist, dass er
die Infusionspumpe (10) in einen Standardmodus versetzt, in dem das untere Okklusionsventil aktiviert ist;
während sich die Infusionspumpe im Standardmodus befindet und das untere Okklusionsventil aktiviert ist, mit dem Unterdrucksensor einen Druck innerhalb des Fluidschlauchs auf eine Schwellenwertabweichung von einem erwarteten Druck überwacht;
vor dem Empfang eines Signals zum Starten einer Infusion eines Fluids durch den Fluidschlauch zu bestimmen, ob die Schwellenwertabweichung erfasst wurde;
die Infusionspumpe so zu konfigurieren, dass sie die Infusion startet, wenn das Signal empfangen wird, nachdem die Schwellenwertabweichung erfasst wurde; und
eine Benachrichtigung bereitzustellen, ohne die Infusion zu starten, wenn das Signal empfangen wird und die Schwellenwertabweichung nicht erfasst wird.

14. Die Infusionspumpe nach Anspruch 13, wobei der Prozessor ferner konfiguriert ist, um:
das Signal zum Starten der Infusion des Fluids durch den Fluidschlauch zu empfangen; und
die Infusionspumpe so zu konfigurieren, dass sie die Infusion basierend auf dem Empfang des Signals startet.

15. Die Infusionspumpe nach Anspruch 13 oder Anspruch 14, wobei das Konfigurieren der Infusionspumpe zum Starten der Infusion umfasst:
Anzeigen einer grafischen Startsteuerung auf einem mit der Infusionspumpe verbundenen Bildschirm, die bei Aktivierung die Infusion startet, wobei die grafische Startsteuerung erst angezeigt wird, wenn die Schwellenwertabweichung erkannt wird.

16. Die Infusionspumpe nach einem der Ansprüche 13 bis 15, wobei die Schwellenwertabweichung einen Spannungsspitzenwert in einer Vielzahl von Spannungsmesswerten über einen Zeitraum umfasst.

17. Die Infusionspumpe nach einem der Ansprüche 13 bis 15, wobei das Überwachen des Drucks auf die Schwellenwertabweichung umfasst:
Bestimmen einer Steigung der Spannungsmesswerte; und
Bestimmen, dass die Steigung einen Schwellenwert für die Steigung erfüllt.

18. Die Infusionspumpe nach einem der Ansprüche 13 bis 17, wobei der Prozessor ferner so konfiguriert ist, dass er
im Standardmodus Überwachen eines oberen Drucksensors der Infusionspumpe auf eine obere Druckabweichung in einem stromaufwärts gelegenen Abschnitt des Fluidschlauchs, wobei der obere Drucksensor stromaufwärts von einem oberen Okklusionsventil des einen oder der mehreren Okklusionsventile angeordnet ist; und
Bestimmen auf der Grundlage des Empfangens des Signals zum Starten der Infusion, ob die obere Druckabweichung erfasst wurde;
Bereitstellen eines Signals, dass das untere Okklusionsventil nicht funktioniert, wenn die obere Druckabweichung nach dem Empfangen des Signals zum Starten der Infusion erkannt wird.

19. Die Infusionspumpe nach Anspruch 18, wobei die Benachrichtigung einen Benutzer anweist, eine mit dem Fluidschlauch gekoppelte Rollenklemme zu überprüfen, und wobei der Prozessor ferner so konfiguriert ist, dass er
ein Signal empfängt, dass die Rollenklemme offen ist;
Bereitstellen einer Aufforderung zum Überprüfen der Platzierung des Fluidschlauchs in der Infusionspumpe;
Empfangen einer Bestätigung der Platzierung des Fluidschlauchs; und
Bereitstellen einer Warnung bezüglich des unteren Okklusionsventils auf der Grundlage des Empfangens der Bestätigung.

20. Die Infusionspumpe nach Anspruch 18, wobei der Prozessor ferner konfiguriert ist, um
ein Signal zum Aufrufen eines Wartungsmodus zu empfangen, in dem das untere Okklusionsventil deaktiviert und ein oberes Okklusionsventil der einen oder mehreren Okklusionsventile aktiviert wird, wobei das Aktivieren des oberen Okklusionsventils ein Zusammendrücken des Fluidschlauchs am oberen Okklusionsventil bewirkt;
ein Signal zu empfangen, dass der Fluidschlauch mit dem Fluid gefüllt ist;
Empfangen eines Signals, dass eine Rollenklemme stromabwärts der Infusionspumpe geöffnet ist;
Bestimmen, nachdem die Rollenklemme geöffnet ist, ob eine obere Druckabweichung durch einen oberen Drucksensor stromaufwärts des oberen Okklusionsventils erfasst wird; und
Bereitstellen einer Benachrichtigung bezüglich eines Ausfalls des oberen Okklusionsventils, wenn die obere Druckabweichung erfasst wird.

## Revendications

1. Support de stockage non transitoire lisible par une machine, contenant des instructions qui, lorsqu'elles sont exécutées par une ou plusieurs unités de traitement, réalisent un processus, comprenant :
faire fonctionner une pompe à perfusion (10) avec une ou plusieurs valves d'occlusion comprenant une valve d'occlusion inférieure, la valve d'occlusion inférieure configurée pour, lorsqu'elle est activée, provoquer une compression d'une tubulure de fluide (20) chargée dans la pompe à perfusion (10), la compression isolant fluidiquement une partie en aval de la tubulure de fluide d'une partie en amont de la tubulure de fluide lorsque la valve d'occlusion inférieure fonctionne dans des conditions de fonctionnement normales ;
placer la pompe à perfusion (10) dans un mode par défaut dans lequel la valve d'occlusion inférieure est activée ;
lorsque le mode par défaut est activé et que la valve d'occlusion inférieure est activée, un capteur de pression inférieure (82) surveille la pression à l'intérieur de la tubulure de fluide pour détecter un écart de seuil par rapport à la pression attendue ;
déterminer, avant de recevoir l'indication de commencer la perfusion d'un fluide dans la tubulure de fluide (20), si l'écart de seuil a été détecté ;
configurer la pompe à perfusion (10) pour commencer la perfusion lorsque l'indication est reçue après la détection de l'écart de seuil ; et
fournir une notification sans commencer la perfusion lorsque l'indication est reçue et que l'écart de seuil n'est pas détecté.

2. Le support de stockage non transitoire lisible par une machine de la revendication 1, le processus comprenant en outre :
recevoir l'indication de commencer la perfusion du fluide dans la tubulure de fluide; et
configurer la pompe à perfusion pour commencer la perfusion en fonction de la réception de l'indication.

3. Le support de stockage non transitoire lisible par une machine de la revendication 1 ou de la revendication 2, dans lequel la configuration de la pompe à perfusion pour commencer la perfusion comprend :
présenter, pour affichage sur un écran associé à la pompe à perfusion, une commande de commencement graphique qui, lorsqu'elle est activée, entraîne le commencement de la perfusion, la commande de commencement graphique n'étant pas affichée tant que l'écart de seuil n'est pas détecté.

4. Le support de stockage non transitoire lisible par une machine de l'une des revendications 1 à 3, dans lequel l'écart de seuil comprend une pointe de tension dans une pluralité de lectures de tension sur une période de temps.

5. Le support de stockage non transitoire lisible par une machine de l'une des revendications 1 à 4, dans lequel la surveillance de la pression pour l'écart de seuil comprend :
déterminer une pente des relevés de tension ; et
déterminer que la pente est conforme à une pente seuil.

6. Le support de stockage non transitoire lisible par une machine de l'une des revendications 1 à 4, dans lequel la surveillance de la pression pour l'écart de seuil comprend :
déterminer qu'au moins une lecture de la pluralité des lectures de tension satisfait à une tension seuil.

7. Le support de stockage non transitoire lisible par une machine de l'une des revendications 1 à 6, le processus comprenant en outre :
en mode par défaut, surveiller un capteur de pression supérieure de la pompe à perfusion pour détecter un écart de pression supérieure dans une partie amont de la tubulure de fluide, le capteur de pression supérieure étant en amont d'une valve d'occlusion supérieure de la ou des valves d'occlusion ; et
déterminer, sur la base de la réception de l'indication de commencer la perfusion, si l'écart de pression supérieur a été détecté ;
fournir une indication que la valve d'occlusion inférieure ne fonctionne pas lorsque l'écart de pression supérieur est détecté après avoir reçu l'indication de commencer la perfusion.

8. Le support de stockage non transitoire lisible par une machine de l'une des revendications 1 à 7, dans lequel la notification indique une occlusion dans la tubulure de fluide.

9. Le support de stockage non transitoire lisible par une machine de l'une des revendications 1 à 8, dans lequel la notification demande à l'utilisateur de vérifier une pince à rouleaux couplée à la tubulure de fluide.

10. Le support de stockage non transitoire lisible par une machine de la revendication 9, le processus comprenant en outre :
recevoir une indication que la pince à rouleaux est ouverte ;
fournir une demande de vérification de l'emplacement de la tubulure de fluide dans la pompe à perfusion ;
recevoir la confirmation de la mise en place de la tubulure de fluide ; et
fournir, sur la base de la réception de la confirmation, une alerte concernant la valve d'occlusion inférieure.

11. Le support de stockage non transitoire lisible par une machine de l'une des revendications 1 à 8, le processus comprenant en outre :
recevoir une indication pour entrer dans un mode de maintenance dans lequel la valve d'occlusion inférieure est désactivée et une valve d'occlusion supérieure d'une ou plusieurs valves d'occlusion est activée, la valve d'occlusion supérieure étant activée comprend la compression de la tubulure de fluide au niveau de la valve d'occlusion supérieure ;
recevoir une indication selon laquelle la tubulure de fluide est amorcée avec le fluide ;
recevoir une indication qu'une pince à rouleaux en aval de la pompe à perfusion est ouverte ;
déterminer, après l'ouverture de la pince à rouleaux, si un écart de pression supérieure est détecté par un capteur de pression supérieure, en amont de la valve d'occlusion supérieure ; et
fournir une notification concernant la défaillance de la valve d'occlusion supérieure lorsque la déviation de la pression supérieure est détectée.

12. Le support de stockage non transitoire lisible par une machine de l'une des revendications 1 à 8, dans lequel l'écart de seuil est provoqué par l'ouverture d'une pince à rouleaux en aval de la pompe à perfusion pour ouvrir un chemin de fluide entre la pompe à perfusion et un patient auquel la tubulure de fluide est connectée fluidiquement pour la perfusion d'un fluide par la pompe à perfusion, l'ouverture de la pince à rouleaux se produisant après que la tubulure de fluide a été amorcée avec le fluide avant que la pompe à perfusion ne soit placée en mode par défaut.

13. Pompe à perfusion (10), comprenant :
une ou plusieurs valves d'occlusion comprenant une valve d'occlusion inférieure, la valve d'occlusion inférieure configurée pour, lorsqu'elle est activée, provoquer une compression d'une tubulure de fluide (20) chargée dans la pompe à perfusion (10), la compression isolant fluidiquement une partie en aval de la tubulure de fluide d'une partie en amont de la tubulure de fluide lorsque la valve d'occlusion inférieure fonctionne dans des conditions de fonctionnement normales ;
un capteur de pression inférieure (82) en aval de la valve d'occlusion inférieure ;
un processeur configuré pour :
placer la pompe à perfusion (10) dans un mode par défaut dans lequel la valve d'occlusion inférieure est activée ;
lorsque le mode par défaut est activé et que la valve d'occlusion inférieure est activée, surveiller, à l'aide du capteur de pression inférieur, une pression à l'intérieur de la tubulure de fluide pour détecter un écart de seuil par rapport à une pression attendue ;
déterminer, avant de recevoir l'indication de commencer la perfusion d'un fluide dans la tubulure de fluide, si l'écart de seuil a été détecté ;
configurer la pompe à perfusion pour qu'elle commence la perfusion lorsque l'indication est reçue après la détection de l'écart de seuil ; et
fournir une notification sans commencer la perfusion lorsque l'indication est reçue et que l'écart de seuil n'est pas détecté.

14. La pompe à perfusion de la revendication 13, dans laquelle le processeur est en outre configuré pour :
recevoir l'indication de commencer la perfusion du fluide dans la tubulure de fluide ; et
configurer la pompe à perfusion pour qu'elle commence la perfusion en fonction de la réception de l'indication.

15. La pompe à perfusion de la revendication 13 ou de la revendication 14, dans laquelle la configuration de la pompe à perfusion pour commencer la perfusion comprend :
présenter, pour affichage sur un écran associé à la pompe à perfusion, une commande de commencement graphique qui, lorsqu'elle est activée, entraîne le commencement de la perfusion, la commande de commencement graphique n'étant pas affichée tant que l'écart de seuil n'est pas détecté.

16. La pompe à perfusion de l'une des revendications 13 à 15, dans laquelle l'écart de seuil comprend un pic de tension dans une pluralité de lectures de tension sur une période de temps.

17. La pompe à perfusion de l'une des revendications 13 à 15, dans laquelle la surveillance de la pression pour l'écart de seuil comprend :
déterminer une pente des relevés de tension ; et
déterminer que la pente est conforme à une pente seuil.

18. La pompe à perfusion de l'une des revendications 13 à 17, dans laquelle le processeur est en outre configuré pour :
en mode par défaut, surveiller un capteur de pression supérieure de la pompe à perfusion pour détecter un écart de pression supérieure dans une partie amont de la tubulure de fluide, le capteur de pression supérieure étant en amont d'une valve d'occlusion supérieure de la ou des valves d'occlusion ; et
déterminer, sur la base de la réception de l'indication de commencer la perfusion, si l'écart de pression supérieur a été détecté ;
fournir une indication que la valve d'occlusion inférieure ne fonctionne pas lorsque l'écart de pression supérieur est détecté après avoir reçu l'indication de commencer la perfusion.

19. La pompe à perfusion de la revendication 18, dans laquelle la notification demande à un utilisateur de vérifier une pince à rouleaux couplée à la tubulure de fluide, et dans laquelle le processeur est en outre configuré pour :
recevoir une indication que la pince à rouleaux est ouverte ;
fournir une demande de vérification de l'emplacement de la tubulure de fluide dans la pompe à perfusion ;
recevoir la confirmation de la mise en place de la tubulure de fluide ; et
fournir, sur la base de la réception de la confirmation, une alerte concernant la valve d'occlusion inférieure.

20. La pompe à perfusion de la revendication 18, dans laquelle le processeur est en outre configuré pour :
recevoir une indication pour entrer dans un mode de maintenance dans lequel la valve d'occlusion inférieure est désactivée et une valve d'occlusion supérieure parmi une ou plusieurs valves d'occlusion est activée, l'activation de la valve d'occlusion supérieure consiste à provoquer une compression de la tubulure de fluide au niveau de la valve d'occlusion supérieure ;
recevoir une indication selon laquelle la tubulure de fluide est amorcée avec le fluide ;
recevoir une indication qu'une pince à rouleaux en aval de la pompe à perfusion est ouverte ;
déterminer, après l'ouverture de la pince à rouleaux, si un écart de pression supérieure est détecté par un capteur de pression supérieure, en amont de la valve d'occlusion supérieure ; et
fournir une notification concernant la défaillance de la valve d'occlusion supérieure lorsque la déviation de la pression supérieure est détectée.
